# EUROPÄISCHE PATENTSCHRIFT

(11) **EP 1 508 558 B1**
(45) Veröffentlichungstag und Bekanntmachung des Hinweises auf die Patenterteilung: **24.10.2012**
(21) Anmeldenummer: 04103134.5
(22) Anmeldetag: 02.07.2004
(51) Int. Cl.: C07C 29/04, C07C 31/12

(54) **Verfahren zur Erzeugung von tert.-Butanol**
Process for the preparation of tert.-butanol
Procédé pour la préparation de tert.-butanol

(30) Priorität: 22.08.2003 DE 10338581
(43) Veröffentlichungstag der Anmeldung: 23.02.2005
(73) Patentinhaber: Evonik Oxeno GmbH, 45772 Marl (DE)
(72) Erfinder: Santiago Fernandez, Silvia, 46049 Oberhausen (DE); Beckmann, Andreas Dr., 45657, Recklinghausen (DE); Nierlich, Franz Dr., 45768 Marl (DE); Reusch, Dieter Dr., 45772 Marl (DE)
(74) Vertreter: Hirsch, Hans-Ludwig

(56) Entgegenhaltungen:
- EP-A- 1 431 264
- DE-A- 2 538 036
- US-A- 4 284 831
- US-A- 4 327 231
- US-A- 6 111 148

## Beschreibung

Die Erfmdung betrifft ein Verfahren zur Herstellung von tert.-Butanol (TBA) durch Umsetzung von Isobuten-haltigen Kohlenwasserstoffgemischen mit Wasser an sauren festen Katalysatoren in mehreren Reaktoren, wobei zur Erhöhung des Umsatzes vor dem letzten Reaktor ein Teil des im Reaktionsgemisch vorhandenen TBA abgetrennt wird.

Tert.-Butanol (TBA) ist ein wichtiges großtechnisch hergestelltes Produkt und wird als Lösungsmittel und als Zwischenprodukt für die Herstellung von Methacrylsäuremethylester verwendet. Es ist Vorstufe für die Herstellung von Peroxiden, wie Peroxiketale, Perester oder Dialkylperoxide, mit mindestens einer tertiären Butylgruppe. Diese Verbindungen werden als Oxidationsmittel und als Starter für Radikalreaktionen, wie beispielsweise Olefinpolymerisation oder Vernetzung von Kunststoffen, eingesetzt. Als Zwischenstufe dient tert.-Butanol zur Gewinnung von reinem Isobuten aus Isobutengemischen. Darüber hinaus ist es ein Reagens zur Einführung von tertiären Butylgruppen. Seine Alkalisalze sind starke Basen, die in vielen Synthesen Verwendung fmden.

Tertiäres Butanol kann durch sauer katalysierte Anlagerung von Wasser an Isobuten erhalten werden. Technische Isobutengemische enthalten häufig auch andere Olefine, wie z. B. 2-Butene. Technische Verfahren wenden daher Bedingungen an, bei denen fast ausschließlich das Isobuten, nicht aber die anderen Olefine hydratisiert werden und Nebenreaktionen, wie Homo- oder Heterooligomerisierung der Olefine, nahezu vollständig unterdrückt werden. Solche Prozesse arbeiten gewöhnlich in der flüssigen Phase und können in zwei Gruppen unterteilt werden: a) Verfahren, bei denen die Umsetzung in einer wässrigen Katalysatorlösung erfolgt und b) heterogene katalytische Prozesse, bei denen feste, in der Reaktionsphase unlösliche Katalysatoren eingesetzt werden.

Die Hydratisierung von Isobuten zu tert.-Butanol mit Hilfe fester, weder in den Edukten noch in den Produkten löslicher, saurer Katalysatoren hat den Vorteil, dass das Reaktionsgemisch säurefrei ist und ohne Verluste durch Rückspaltung oder durch andere Nebenreaktionen zu tert.-Butanol aufgearbeitet werden kann. Die Reaktion läuft an der Oberfläche des Katalysators ab. Damit eine Reaktion zustande kommt, müssen beide Reaktionspartner zur gleichen Zeit an der aktiven Stelle des Katalysators sein. Dies wird dadurch erschwert, das Wasser und Isobuten bzw. ein Isobuten-haltiges Kohlenwasserstoffgemisch nicht miteinander mischbar sind. Um akzeptable Umsätze zu erhalten, werden Solventien verwendet, die ein homogenes Gemisch aus Wasser und Isobuten-Einsatzgemisch ermöglichen.

In DE 30 31 702 A1 wird Methanol für diesen Zweck als Lösungsmittel sowohl für Wasser als auch für Isobuten bzw. für ein Isobuten-haltiges Kohlenwasserstoffgemisch beschrieben. Als Produkte werden tert.-Butanol und Methyl-tert.-butylether nebeneinander erhalten.

In EP 0 010 993 A1 werden aliphatische Carbonsäuren mit 1 bis 6 C-Atomen als Lösungsmittel für beide Edukte eingesetzt. Dabei entstehen als Nebenprodukte die tertiären Butylester dieser Säuren. Diese müssen zu tert.-Butanol und Carbonsäuren hydrolisiert werden.

In DE 30 31 702 A1 werden Sulfolane und in US 4 327 231 mehrwertige Alkohole des Neo-Typs, wie beispielsweise Neopentylglykol, eingesetzt. Diese Lösungsmittel müssen vom tert.-Butanol abgetrennt werden. Darüber hinaus besteht die Gefahr, dass das verwendete Lösungsmittel im Dauerbetrieb einer solchen Anlage zersetzt wird.

Um diese Nachteile zu vermeiden, wird in einigen Verfahren das Zielprodukt, TBA, als Lösevermittler eingesetzt. Solche Verfahren sind beispielsweise in WO 99/33775 oder DE 30 25 262 beschrieben. Dabei wird ein Gemisch aus einer Kohlenwasserstofffraktion, die Isobuten enthält, TBA und Wasser in einer Reaktorkaskade an sauren im Festbett angeordneten Katalysatoren umgesetzt. Der erste Reaktor wird meistens in Schlaufenfahrweise und die anderen im geraden Durchgang betrieben. Vor jedem weiteren Reaktor kann Reaktionswasser zudosiert werden. Der Austrag des letzten Reaktors wird destillativ in ein Kohlenwasserstoffgemisch mit dem nicht umgesetzten Isobuten und Roh-TBA getrennt. Ein Teil des Roh-TBA wird in den ersten Reaktor zurückgeführt. Der andere Teil kann als solcher genutzt werden oder auf TBA und/oder TBA/Wasser-Azeotrop aufgearbeitet werden.

Bei diesen Verfahren nimmt von Reaktor zu Reaktor aufgrund des Reaktionsfortschritts der TBA-Gehalt zu und der Isobutengehalt ab. Die Zusammensetzung des Reaktionsgemisch nähert sich dabei mit abnehmender Geschwindigkeit dem thermodynamischen Gleichgewicht zwischen Wasser, Isobuten und TBA, sodass kein vollständiger Umsatz erreicht werden kann. Ausgehend von technischen Isobutenströmen, beispielsweise Raffinat I, werden nur Umsätze von ca. 82 % erreicht.

In der noch nicht offengelegten Anmeldung DE 102 60 991 wird ein Verfahren zur Herstellung von TBA beschrieben, bei dem eine relativ aufwändige Reaktivdestillation eingesetzt wird.

Es bestand vor diesem Hintergrund die Aufgabe, die Wirtschaftlichkeit des Verfahrens zur TBA Herstellung zu verbessern.

Es wurde nun überraschenderweise gefunden, dass die Umsätze an Isobuten zu TBA erhöht und somit auch die Wirtschaftlichkeit verbessert werden kann, wenn das Verfahren in zumindest zwei hintereinandergeschalteten Reaktoren durchgeführt wird, wobei aus dem Reaktionsaustrag des vorletzten Reaktors ein Teil des TBA abgetrennt wird, bevor dieses in den letzten Reaktor eingespeist wird.

Gegenstand der Erfindung ist demnach ein Verfahren zur Herstellung von tert. Butanol (TBA) durch Umsetzung eines Gemisches, das Wasser, TBA und ein Isobuten-haltigen Kohlenwasserstoffgemisch aufweist, an einem sauren Katalysator in einer Reaktorkaskade, die zumindest zwei Reaktoren aufweist, bei 30 bis 120 °C, und anschließende destillative Abtrennung der nicht umgesetzten Kohlenwasserstoffe, welches dadurch gekennzeichnet ist, dass aus dem Reaktoraustrag zumindest eines Reaktors, der nicht der letzte der Reaktorkaskade ist, ein Teil des TBA abgetrennt wird, bevor der verbleibende Rest des an TBA abgereicherten Reaktoraustrags in den nachfolgenden Reaktor geleitet wird.

In technischen Verfahren zur Herstellung von TBA sinkt aufgrund des Reaktionsfortschritts im Reaktionsgemisch von Reaktor zu Reaktor die Konzentration an Isobuten und die Konzentration an TBA nimmt zu. Mit von Reaktor zu Reaktor abnehmender Geschwindigkeit nähert sich die Zusammensetzung des Reaktionsgemisches dem thermodynamischen Gleichgewicht zwischen Isobuten, Wasser und TBA. Um die Reaktionsgeschwindigkeit und somit die Raum-Zeit-Ausbeute zu erhöhen und einen höheren Umsatz des Isobutens zu erreichen, wird im erfindungsgemäßen Verfahren die Konzentration des Isobutens vergrößert und die Konzentration an TBA verringert. Dies geschieht dadurch, dass ein Teil des TBA aus dem Reaktionsaustrag eines Reaktors abgetrennt wird und das an TBA-verarmte Reaktionsgemisch in den nächsten Reaktor, vorzugsweise den letzten eingeleitet wird. Das erfindungsgemäße Verfahren hat den Vorteil, dass deutlich höhere Umsätze im Vergleich zu herkömmlichen Verfahren erzielt werden. Durch die höheren Umsätze wird das Verfahren zudem wirtschaftlicher. Ein ganz besonderer Vorteil kann erzielt werden, wenn zur Verarmung des Reaktoraustrags an TBA dieser ganz oder teilweise in die Roh-TBA-Kolonne gefahren wird und diese zur Abreicherung an TBA genutzt wird, wobei ein Teil des Kopfprodukts der Roh-TBA-Kolonne als an TBA abgereicherter Produktstrom und gegebenenfalls ein Teil des Sumpfs der Kolonne in den nachfolgenden Reaktor gefahren wird. Bei dieser Verfahrensweise kann auf eine zusätzliche Kolonne zur Abreicherung des Reaktorsaustrags des nicht letzten Reaktors der Reaktorkaskade an TBA verzichtet werden. Auf diese Weise lassen sich die Investitionskosten reduzieren und damit die Wirtschaftlichkeit erhöhen.

Das erfindungsgemäße Verfahren wird nachfolgend beschrieben. Sind im nachfolgenden Text Bereiche bzw. Vorzugsbereiche angegeben, so sollen auch alle in diesen Bereichen liegenden, theoretisch möglichen Teilbereiche zum Offenbarungsgehalt der vorliegenden Erfindung gehören, ohne dass diese aus Gründen der besseren Übersichtlichkeit explizit genannt werden.

Das erfmdungsgemäße Verfahren zur Herstellung von TBA durch Umsetzung eines Gemisches, das Wasser, TBA und ein Isobuten-haltigen Kohlenwasserstoffgemisch aufweist, an einem sauren Katalysator in einer Reaktorkaskade, die zumindest zwei Reaktoren aufweist, bei 30 bis 120 °C, und anschließende destillative Abtrennung der nicht umgesetzten Kohlenwasserstoffe, zeichnet sich dadurch aus, dass aus dem Reaktoraustrag zumindest eines Reaktors, vorzugsweise des vorletzten, der nicht der letzte der Reaktorkaskade ist, ein Teil des TBA abgetrennt wird, bevor der an TBA abgereicherte Reaktoraustrag in den nachfolgenden Reaktor, vorzugsweise den letzten Reaktor, geleitet wird.

Vorzugsweise erfolgt die Abtrennung eines Teils des TBA aus dem Reaktionsaustrag des Reaktors, der nicht der letzte der Reaktorkaskade ist, durch Destillation bzw. Rektifikation, wobei der an TBA abgereicherte Produktstrom in den nachfolgenden Reaktor geführt wird und der an TBA angereicherte Produktstrom zusammen mit dem Austrag des nachfolgenden Reaktors in eine Kolonne zur Aufarbeitung von Roh-TBA (Roh-TBA-Kolonne) eingespeist wird. Die Abtrennung eines Teils des TBA kann durch Flashung/einfache Verdampfung oder einfache Destillation erfolgen.

Im erfindungsgemäßen Verfahren erfolgt die TBA-Abtrennung zwischen zwei Reaktoren insbesondere dann, wenn der zwischen den beiden Reaktoren gelegenen Reaktoraustrag eine Isobutenkonzentration kleiner als 15 Massen-%, insbesondere kleiner als 12 Massen-% aufweist oder eine TBA-Konzentration von größer 40 Massen-%, insbesondere größer 45 Massen-% aufweist. Üblicherweise korrelieren diese Werte miteinander. Dies ist aber nicht zwingend notwendig. Besonders bevorzugt erfolgt die Abtrennung des TBA aus einem Reaktorsaustrag zumindest eines Reaktors, der nicht der letzte der Reaktorkaskade ist, der einen Gehalt an Isobuten von kleiner 15 Massen-% aufweist. Durch die TBA-Abtrennung wird die TBA-Konzentration im Zulauf des nächsten Reaktors, vorzugsweise des letzten, auf Werte von 10 bis 50 Massen-%, insbesondere von 20 bis 45 Massen-% und besonders bevorzugt von 25 bis 40 Massen-% gebracht. Die Abtrennung des TBA erfolgt also vorzugsweise so, dass das an TBA abgereicherte Produkt, welches in den nachfolgenden oder letzten Reaktor geführt wird, einen Gehalt an TBA von 10 bis 50 Massen-%, insbesondere von 20 bis 45 Massen-% und besonders bevorzugt von 25 bis 40 Massen-% aufweist.

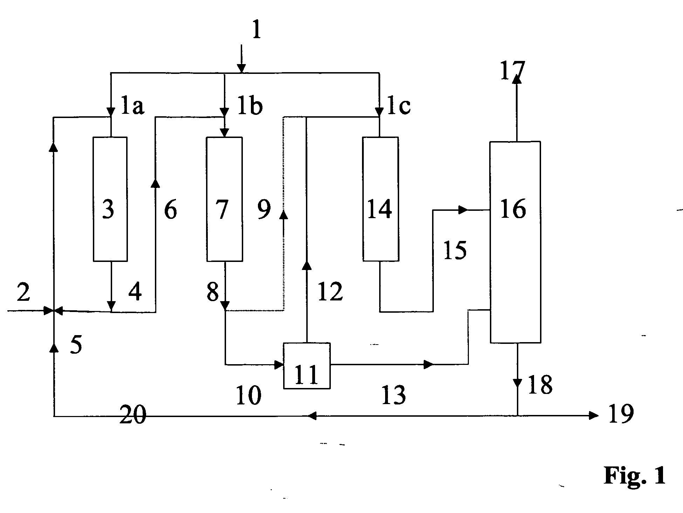

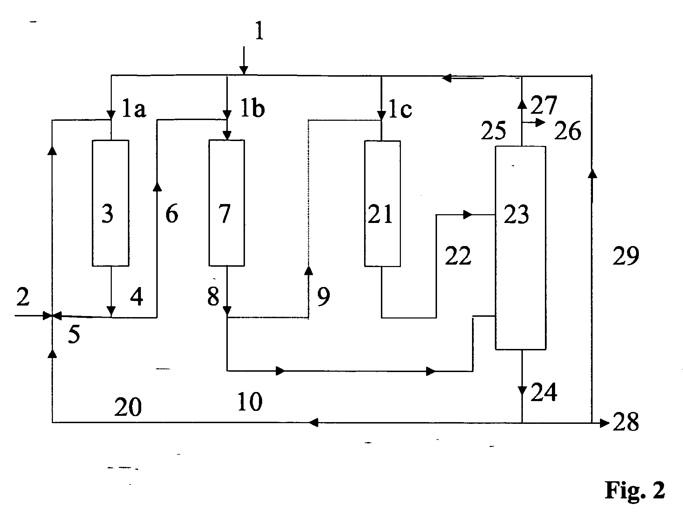

Die TBA-Abtrennung erfolgt im erfmdungsgemäßen Verfahren vorzugsweise durch Destillation. Dabei sind mehrere Ausführungsarten möglich. Zwei Ausführungsarten des erfindungsgemäßen Verfahrens werden beispielhaft in Fig. 1 und Fig. 2 dargestellt. Bei der ersten, bevorzugten Ausführungsart (Fig. 1) wird der gesamte Austrag eines Reaktors, vorzugsweise des vorletzten, oder ein Teil davon in ein an TBA abgereichertes Kopfprodukt, das vorzugsweise die leichtsiedenden Kohlenwasserstoffe mit dem nicht umgesetzten Isobuten sowie Wasser und TBA enthält, und in ein an TBA angereichertes Sumpfprodukt, das vorzugsweise im Wesentlichen TBA, geringe Mengen an C₄-Kohlenwasserstoffen, Wasser und hochsiedende Nebenprodukte aufweist, getrennt. Das Kopfprodukt wird mit zusätzlichem Wasser und gegebenenfalls mit nicht destilliertem Reaktoraustrag in den nächsten Reaktor, vorzugsweise den letzten, eingeleitet. Das Sumpfprodukt kann in eine Trennkolonne (wie z.B. Apparat 16 in Fig. 1) eingespeist werden oder einer anderen Aufarbeitung zugeführt werden.

Da das Kopfprodukt, das in den nächsten Reaktor eingespeist wird, als Lösevermittler für Wasser ebenfalls TBA enthalten sollte, ist bei der Destillation schon eine Trennstufenzahl von 0,3 bis 1 ausreichend. Die destillative Auftrennung kann daher zweckmäßig als Flashung/einfache Verdampfung durchgeführt werden. Auf diese Weise kann beispielsweise erreicht werden, dass aus einem Strom mit nahezu 50 Massen-% an TBA der TBA-Gehalt im Kopfprodukt auf Werte von ca. 35-Massen-% abgesenkt werden kann.

Die Verdampfung erfolgt entweder beim Druck des vorletzten Reaktors, d.h. im Bereich von 7 bis 14 bar_{abs}. oder bei abgesenktem Druck im Bereich von 4 bis 8 bar_{abs.}. Die Wahl des Drucks hängt im Wesentlichen von den Möglichkeiten zur Wärmerückgewinnung in der Anlage ab. Als Verdampfer lassen sich übliche Apparate wie Zwangsumlauf- oder Naturumlaufverdampfer, oder beliebige andere stehende oder liegende Behälter mit Heizvorrichtung verwenden. Das Kopfprodukt tritt meist dampfförmig aus dem Verdampfer und muss anschließend kondensiert werden und dann in den Reaktor geleitet werden oder aber im Reaktoreintritt kondensiert werden, damit die Reaktion in der flüssigen Phase ablaufen kann.

In der zweiten Ausführungsart (Fig. 2) des erfmdungsgemäßen Verfahrens wird der Reaktionsaustrag eines Reaktors, der nicht der letzte der Reaktorkaskade ist, vorzugsweise des vorletzten Reaktors, ganz oder teilweise zusammen mit dem Reaktionsaustrag des letzten Reaktors in eine Kolonne, insbesondere eine Kolonne zur Abtrennung von Roh-TBA (Roh-TBA-Kolonne, Apparat 23 in Fig. 2) geleitet. Ein Teil des an TBA abgereicherten Kopfproduktes dieser Kolonne, das Kohlenwasserstoffe mit dem nicht umgesetzten Isobuten enthält und ein Teil des an TBA angereicherten Sumpfproduktes dieser Kolonne (Roh-TBA) wird unter Zusatz von Reaktionswasser und gegebenenfalls eines Teilstroms eines Reaktoraustrag in einen nachfolgenden Reaktor, vorzugsweise den letzten Reaktor, eingespeist.

Die beiden Ausführungsarten des erfindungsgemäßen Verfahrens sind zur Verdeutlichung der Erfindung als Blockschema in Fig. 1 und Fig. 2 beispielhaft dargestellt. Diese Schemata weisen jeweils drei Reaktoren auf. Es versteht sich von selbst, dass das erfindungsgemäße Verfahren zur Herstellung von TBA auch unter Verwendung von zwei, drei oder mehr als drei Reaktoren durchgeführt werden kann.
In der ersten Ausführungsform des erfmdungsgemäßen Verfahrens gemäß Fig. 1 werden Reaktionswasser (1a), Isobuten-haltiges Einsatzkohlenwasserstoffgemisch (2), Roh-TBA (20) und ein Teil (5) des Reaktionsaustrags (4) aus dem Reaktor (3) in den Reaktor (3) eingeleitet. Ein Teil (6) des Reaktionsaustrages (4) wird zusammen mit Reaktionswasser (1b) im zweiten Reaktor (7) umgesetzt. Der gesamte Austrag (8) aus Reaktor (7) oder ein Teil (10) davon wird in der Destillations- oder Verdampfereinheit (11) in ein Isobuten-armes bzw. praktisch Isobutenfreies Sumpfprodukt (13) und in ein Isobuten-reiches Kopfprodukt (12) getrennt. Das Sumpfprodukt (13) wird in die Destillationskolonne (16) gefahren. Das Kopfprodukt (12) wird zusammen mit Reaktionswasser (1c) und gegebenenfalls mit undestilliertem Austrag (9) in den dritten Reaktor (14) eingespeist. Der Austrag (15) des Reaktors (14) wird zusammen mit Strom (13) in der Kolonne (16) in ein Kopfprodukt (17), das Isobuten und andere leichtsiedende Kohlenwasserstoffe enthält, und in Roh-TBA (18) getrennt. Ein Teil (20) des Roh-TBA wird in den Reaktor (3) zurückgefahren. Der andere Teil (19) kann in einer nicht dargestellten Anlage in Rein-TBA und/oder TBA/Wasser-Azeotrop aufgearbeitet werden.

Eine mögliche Verschaltung von Apparaten und Strömen gemäß der zweiten Ausführungsform des erfindungsgemäßen Verfahrens zeigt Fig. 2. Reaktionswasser (1a), Isobuten-haltiges Einsatzkohlenwasserstoffgemisch (2), Roh-TBA (20) und ein Teil (5) des Austrags (4) aus dem Reaktor (3) werden in den Reaktor (3) eingeleitet. Ein Teil (6) des Austrages (4) wird zusammen mit Reaktionswasser (1b) im zweiten Reaktor (7) umgesetzt. Der Austrag (8) des Reaktors (7) wird ganz oder ein Teil (10) davon in die Destillationskolonne (23) geleitet. Dort erfolgt die Auftrennung in ein Kopfprodukt (25), das leicht siedende Kohlenwasserstoffe mit dem nicht vollständig umgesetzten Isobuten enthält, und Roh-TBA (24). Ein Teil (27) des Kopfproduktes (25) wird optional zusammen mit einem Teil (29) des Roh-TBA (24) und Reaktionswasser (1c) und gegebenenfalls mit einem Teil (9) des Austrags (8) vom zweiten Reaktor (7) in den dritten Reaktor (21) geleitet. Der Austrag (22) des dritten Reaktors (21) wird in die Destillationskolonne (23) eingespeist. Der zweite Teil (20) des Roh-TBA (24) wird in den ersten Reaktor (3) zurückgeführt. Der dritte Teil (28) des Roh-TBA kann in einer nicht gezeichneten Anlage auf reines TBA und/oder TBA/Wasser-Azeotrop aufgearbeitet werden. Der Kohlenwasserstrom (26) wird aus dem Verfahren ausgeschleust.

Als Ausgangsstoff kann in dem erfindungsgemäßen Verfahren ein Isobuten-haltiges Kohlenwasserstoffgemisch, oder optional auch reines Isobuten eingesetzt werden. Bevorzugt enthält das Isobuten-haltige Kohlenwasserstoffgemisch keine Acetylenderivate, weniger als 5000 ppm an Dienen und keine weiteren Olefine mit einer oder zwei Verzweigung(en) an der olefinischen Doppelbindung.

Technische Gemische, die Isobuten enthalten, sind beispielsweise Leichtbenzinfraktionen aus Raffinerien, C₄-Fraktionen aus FCC-Einheiten oder Steamcrackern, Gemische aus Fischer-Tropsch-Synthesen, Gemische aus Dehydrierung von Butanen, Gemische aus Skelettisomerisierung linearer Butene, Gemische, entstanden durch Metathese von Olefmen oder anderen technischen Prozessen.

Diese Gemische werden ggf. nach Entfernung der mehrfach ungesättigten Verbindungen im erfindungsgemäßen Verfahren eingesetzt. Beispielsweise kann die Gewinnung eines geeigneten Isobutengemisches aus der C₄-Fraktion eines Steamcrackers durch Extraktion des Butadiens oder durch dessen Selektivhydrierung zu linearen Butenen erfolgen. Dieser Einsatzstoff (Raffinat I bzw. selektivhydriertes Crack-C₄) besteht aus n-Butan, Isobutan, den drei linearen Butenen und Isobuten und ist ein bevorzugtes Edukt für das erfindungsgemäße Verfahren. Der Isobuten-Gehalt im bevorzugt eingesetzten Raffinat I liegt typischerweise im Bereich von 30 bis 60%.

Optional kann Raffinat I, hydriertes Crack-C₄ oder ein ähnlich zusammengesetztes Kohlenwasserstoffgemisch in einer Reaktivkolonne hydroisomerisiert werden. Dadurch kann ein Gemisch aus Isobuten, (und ggf. 1-Buten) und Isobutan gewonnen werden, welches dann als Rohstoff in der erfindungsgemäßen TBA-Synthese (Strom (2) eingesetzt werden kann.

Die Konzentration des Isobutens im Kohlenwasserstoffgemisch kann in weitem Bereich variieren. Es ist jedoch wegen der Wirtschaftlichkeit des Verfahrens bevorzugt, Kohlenwasserstoffgemische mit einer Isobutenkonzentration größer 30 Massen-%, vorzugsweise größer 40 Massen-% einzusetzen.

Als Katalysator wird bevorzugt ein saurer Ionenaustauscher, der weder im Einsatzstoffgemisch noch im Produktgemisch löslich ist, eingesetzt. Der Katalysator darf unter Reaktionsbedingungen weder durch Hydrolyse noch durch andere Reaktionen saure Stoffe an das Produktgemisch abgeben, weil dies zu Ausbeuteverlusten bei der Aufarbeitung des Reaktionsgemischs führen würde.

Für die Aktivität der geeigneten Katalysatoren gilt, dass sie unter Reaktionsbedingungen die Hydratisierung von Isobuten bewirken, jedoch kaum die von nicht verzweigten Olefinen. Weiterhin dürfen sie die Oligomerisierung von Olefinen kaum katalysieren.

Eine geeignete Gruppe von Katalysatoren sind feste Ionenaustauscherharze mit Sulfonsäuregruppen. Besonders geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfongruppen verwendet. Die Harze können gelförmig, makroporös oder schwammförmig hergestellt werden. Geeignete Harze des Styrol-Divinylbenzol-Typs werden u. a. unter folgenden Handelsnamen verkauft: Duolite C20, Duolite C26, Amberlyst 15, Amberlyst 35, Amberlite IR-120, Amberlite 200, Dowex 50, Lewatit SPC 118, Lewatit SPC 108, K2611, OC 1501.

Die Eigenschaften dieser Harze, insbesondere spezifische Oberfläche, Porosität, Stabilität, Quellung bzw. Schrumpfung und Austauschkapazität, können durch den Herstellprozess variiert werden.

Im erfindungsgemäßen Verfahren werden die Ionenaustauscherharze bevorzugt in ihrer H-Form verwendet. Die Ionenaustauscherkapazität beträgt vorzugsweise von 2 bis 7, insbesondere von 3 bis 6 eq/kg (bezogen auf feuchtes handelsübliches Harz). Es werden vorzugsweise makroporöse Harze eingesetzt, wie beispielsweise Amberlyst 15, Amberlyst 35, Lewatit SCP 118, Lewatit SCP 108 oder K2631. Die mittlere Korngröße des Harzes kann bevorzugt von 0,5 bis 2 mm betragen. Die Korngrößenverteilung kann eng oder weit gewählt werden. Bevorzugt werden Ionenaustauscherharze mit sehr einheitlicher Korngröße (monodisperse Harze) eingesetzt. Bei der Verwendung mehrerer Reaktoren können diese mit Harz gleicher oder unterschiedlicher Korngröße (bzw. Korngrößenverteilung) gefüllt sein.

Optional können die Ionenaustauscherharze nicht nur als Kugeln, sondern auch als Formkörper, wie beispielsweise Zylinder, Ringe oder Kugeln, oder auf Formkörper aufpolymerisiert eingesetzt werden.

Es kann vorteilhaft sein, in Reaktoren, die mit hohen Lineargeschwindigkeiten durchströmt werden, zur Verringerung des Differenzdruckes ein Ionenaustauscherharz mit einem größeren Korn einzusetzen, und in Reaktoren, die mit einer geringen Lineargeschwindigkeit durchströmt werden, zur Erzielung des optimalen Umsatzes ein Ionenaustauscherharz mit kleinerem Korn einzusetzen.

Um zu verhindern, dass das Harz während des Betriebs saure Gruppen abspaltet und somit Störungen im Aufarbeitungsteil des Verfahrens verursachen könnte und um eine hohe Aktivität des Katalysators über einen langen Zeitraum beizubehalten, kann das Ionenaustauscherharz vorbehandelt werden, beispielsweise durch Spülen mit Wasser, TBA oder TBA/Wasser-Gemischen vorzugsweise im Temperaturbereich von 40 bis 120 °C.

Da durch die Hydratisierung von Isobuten Wasser verbraucht wird, sinkt der Wassergehalt in der Reaktionsmischung ständig ab. Um eine möglichst hohe Ausbeute und Reaktionsgeschwindigkeit zu erhalten, ist es vorteilhaft Wasser nachzudosieren. Dies könnte beispielsweise dadurch geschehen, dass beispielsweise in einem Rohrreaktor an verschiedenen Stellen Wasser eingespeist wird. In der Praxis ist es jedoch schwierig, genau die erforderliche Wassermenge einzubringen und zu erreichen, dass sofort eine homogene Lösung entsteht. Es ist technisch einfacher und daher vorteilhaft, mehrere Reaktoren in Reihe zu schalten und die notwendige Wassermenge jeweils vor dem Eintritt des Reaktionsgemisches in den Reaktor, also z.B. zwischen den Reaktoren einzuspeisen.

Die Wassermenge im Eduktgemisch stammt aus einer tert.-Butanol-Wasser-Lösung, die, abgesehen von der Anfahrphase, im Prozess selbst nach Abtrennen der Kohlenwasserstoffe anfällt, d. h. durch Rezyklieren eines Teils des Reaktoraustrags. Wenn diese Wassermenge nicht ausreicht, wird zusätzlich Wasser (Reaktionswasser oder auch eine Mischung mit tert.-Butanol) eingespeist. Die Wassermenge im Gemisch, das einem Reaktor zugeführt wird, ist abhängig von dem Verhältnis von TBA zum C₄-Kohlenwasserstoff-Gemisch. Bevorzugt wird maximal soviel Wasser eingesetzt, wie im TBA/C₄-Kohlenwasserstoff-Gemisch löslich ist, so dass eine homogene Lösung vorliegt. Insbesondere werden, bis ggf. auf den letzten Reaktor, Wassergehalte im TBA/C₄-Kohlenwasserstoff-Gemisch eingestellt, die geringer als die jeweilige maximalen Löslichkeiten sind. Ganz besonders werden Wasserkonzentrationen eingestellt, wie sie in der noch nicht veröffentlichten Patentschrift DE 10259413.9, bzw. 10330710.9 beansprucht werden. In die weiteren Reaktoren kann reines Wasser oder auch eine Mischung mit tert.-Butanol eingebracht werden. Es kann auch ein Teil des durch die Umsetzung erhaltenen TBA zur Herstellung eines homogenen Gemisches mit Wasser und dem Isobuten-haltigen Kohlenwasserstoffgemisch rezykliert werden.

Das Verfahren kann in chargenweise oder kontinuierlich arbeitenden Reaktoren, die üblicherweise bei Feststoff/Flüssigkeits-Kontaktreaktionen zum Einsatz gelangen, durchgeführt werden. Bei der Verwendung von kontinuierlich arbeitenden Strömungsreaktoren bedient man sich vorzugsweise, jedoch nicht zwingend, eines Festbetts. Wenn ein Festbett-Strömungsreaktor verwendet wird, kann die Flüssigkeit aufwärts oder abwärts strömen. Meistens wird ein Abwärtsströmen der Flüssigkeit bevorzugt.

Weiterhin ist es möglich, den Reaktor unter Produktrückführung oder im geraden Durchgang zu betreiben.

Bei der Verwendung von Rohrreaktoren kann das Verhältnis von Länge zu Durchmesser der Katalysatorschüttung variiert werden, entweder durch die geometrischen Maße des Reaktors oder durch dessen Füllgrad. Bei gleicher Kontaktmenge und Belastung (LHSV) können somit unterschiedliche Leerrohrgeschwindigkeiten erreicht werden. Reaktoren, bei denen ein Teil des Reaktionsgemisches zurückgeführt wird, können z. B. mit Leerrohrgeschwindigkeiten von 12 bis 60 m/h ganz besonders mit 12 bis 30 m/h betrieben werden. In den Reaktoren, die im geraden Durchgang durchströmt werden, können die Leerrohrgeschwindigkeiten im Bereich von 0,8 bis 55 m/h und besonders im Bereich von 1 bis 25 m/h liegen.

Die Katalysatorbelastung (LHSV) beträgt bei Reaktoren, die unter Produktrückführung betrieben werden, vorzugsweise von 0,3 bis 10 h⁻¹, insbesondere von 1 bis 6 h⁻¹. Bei Reaktoren, die im geraden Durchgang durchströmt werden, liegen die Belastungen vorzugsweise im Bereich von 0,1 bis 5,0 h⁻¹, insbesondere im Bereich von 0,4 bis 3 h⁻¹.

Das erfindungsgemäße Verfahren wird in zumindest zwei hintereinandergeschalteten Reaktoren, die jeweils gleiche oder unterschiedliche Reaktortemperaturen aufweisen können, durchgeführt. Vorzugsweise wird das Verfahren in mehreren, hintereinandergeschalteten Reaktoren, die in Fließrichtung sinkende Temperaturen aufweisen, durchgeführt.

Werden einer oder mehrere der Reaktoren unter Produktrückführung betrieben, so wird vorzugsweise ein Kreislauffaktor (Verhältnis von im Kreis gepumpter Menge zu Frischzulauf) von 0,1 bis 10 eingestellt. Der Kreislauffaktor für den ersten Reaktor beträgt dabei bevorzugt 1 bis 5, insbesondere 2 bis 3,5.

In einer bevorzugten Ausführungsart des erfmdungsgemäßen Verfahrens kann der erste Reaktor unter Produktrückführung und die weiteren Reaktoren im geraden Durchgang betrieben werden. Die Anzahl der verwendeten Reaktoren liegt bevorzugt zwischen 2 und 10, besonders bevorzugt zwischen 2 und 4.

Jeder Reaktor kann adiabatisch oder quasi isotherm, d. h., mit einer Temperaturdifferenz von Reaktorzulauf zu Reaktorausgang von kleiner 10 K, bevorzugt kleiner 5 K und besonders bevorzugt kleiner 1 K betrieben werden. Ein zu hoher Temperaturanstieg ist wegen der ungünstigen Gleichgewichtsbeeinflussung (Rückspaltung) und der Zunahme von Nebenreaktionen zu vermeiden.

Die Temperaturen, bei denen das erfindungsgemäße Verfahren durchgeführt wird, betragen vorzugsweise von 30 bis 120°C. Bei tieferen Temperaturen ist die Reaktionsgeschwindigkeit zu gering und bei höheren treten vermehrt Nebenreaktionen, wie beispielsweise die Oligomerisierung der Olefine auf. Vorzugsweise werden die Reaktoren im Temperaturbereich 35 bis 80 °C betrieben. Die Temperaturen in verschiedenen Reaktoren können gleich oder verschieden innerhalb des angegebenen Bereichs sein. Eine Verfahrensvariante besteht darin, die Temperatur in Flussrichtung von Reaktor zu Reaktor zu senken. Da mit sinkender Temperatur die Gleichgewichtslage günstiger wird, kann somit ein höherer Umsatz erreicht werden. Allerdings ist es nicht sinnvoll, die Temperatur unter 35°C zu senken, da dann die Reaktion für ein technisches Verfahren zu langsam wird.

Beispielsweise kann bei vier hintereinander geschalteten Reaktoren der erste bei einer mittleren Temperatur von 50 bis 70 °C, vorzugsweise von 60 bis 70 °C, der zweite bei einer Temperatur von 50 bis 60 °C, der dritte bei einer Temperatur von 40 bis 50 °C, vorzugsweise von 40 bis 46 °C und der vierte Reaktor bei einer Temperatur von 38 bis 50 °C, vorzugsweise von 38 bis 42 °C betrieben werden.

Die erfindungsgemäße Umsetzung kann bei einem Druck gleich oder über dem Dampfdruck des Einsatz-Kohlenwasserstoffgemisches bei der jeweiligen Reaktionstemperatur durchgeführt werden, vorzugsweise bei einem Druck unter 40 bar. Um in den Reaktoren Verdampfungsprobleme zu vermeiden, sollte der Druck 2 bis 4 bar höher als der Dampfdruck des Reaktionsgemisches sein.

Die folgenden Beispiele sollen die Erfindung erläutern die sich aus der Beschreibung und den Patentansprüchen ergibt.

### Beispiele:

Der für die Versuche 1 bis 3 eingesetzte Raffinatstrom hatte die folgende Zusammensetzung:

| | |
|---|---|
| n-Butan | 8,2 Massen-Teile |
| Iso-Butan | 2,3 Massen-Teile |
| 1-Buten | 29,8 Massen-Teile |
| 2-Buten (cis + trans) | 14,3 Massen-Teile |
| Isobuten | 45,4 Massen-Teile |

Dieser Raffinatstrom wurde in einer Labor-Versuchsanlage mit Wasser und Rück-TBA als Lösungsvermittler umgesetzt. Es wurden 4 Reaktoren hintereinandergeschaltet, wobei der erste Reaktor als Kreislaufreaktor betrieben wurde. Die Reaktoren 1 bis 3 bestanden aus Katalysatorfestbettstufen mit einem Katalysatorvolumen von jeweils 250 bis 450 ml, der vierte Reaktor wurde mit einem Katalysatorvolumen von 950 ml betrieben. Als Katalysator wurde Amberlyst 35 bzw. Amberlyst 15 in H⁺-Form eingesetzt. Die Produktströme wurden gaschromatographisch analysiert. Die Komponenten n-Butan, Iso-Butan, 1-Buten, 2-Buten (cis + trans) wurden in Summe als Rest-C₄ aufgelistet. Die angegebenen Zusammensetzungen bezogen sich auf Produktausträge im quasistationären Gleichgewicht, das nach einer Versuchsdauer von 20 bis 30 Stunden erreicht worden war. Der Druck in der Anlage betrug 12 bar, absolut, die Laborkolonne hatte einen Durchmesser von 50 mm und wurde bei 6 bar, absolut betrieben. Der erste Reaktor wurde mit einer Eintrittstemperatur von 55 °C, alle weiteren 3 Reaktoren mit 50 °C betrieben. Zur besseren Vergleichbarkeit der einzelnen Versuche wurde Strom (20) stets insofern mit zusätzlichem Wasser ergänzt, so dass ein Wassergehalt von 10,4 Massenanteilen in der Mischung eingestellt wurde. Dies entspricht einer erhöhten Prozesswasserzugabe am ersten Reaktor.

### 1. Beispiel (gemäß der Erfindung)

Die Stromnummern in der folgenden Tabelle 1 entsprechen denen in Fig. 1. Für den Verdampfungsschritt (11) wurde ein beheizter Labordruckbehälter verwendet. Der Zulauf an Raffinatstrom (Raffinat I) betrug 0,3 kg/h.

**Tabelle 1**

| Stromnummer | Strombezeichnung (Massenstrom) | Massen-Teile |
|---|---|---|
| 1 | Prozesswasser | 91,3 Wasser |
| | (0,048 kg/h) | 8,7 TBA |
| 9 | Bypass (0 kg/h) | |
| 10 | Vor-Flash | 6, 8 Isobuten |
| | (0,415 kg/h) | 3,9 Wasser |
| | | 49,7 TBA |
| | | 39,5 Rest-C₄ |
| 12 | Flash-Raffinat | 8,9 Isobuten |
| | (0,284 kg/h) | 3,4 Wasser |
| | | 35,1 TBA |
| | | 52,6 Rest-C₄ |
| 13 | Flash-TBA | 2,3 Isobuten |
| | (0,131 kg/h) | 5,2 Wasser |
| | | 81,4 TBA |
| | | 11,1 Rest-C₄ |
| 15 | Schlussreaktor- | 5,2 Isobuten |
| | Ausgang | 2,2 Wasser |
| | (0,284 kg/h) | 40,0 TBA |
| | | 52,6 Rest-C₄ |
| 20 | Rück-TBA | 10,4 Wasser |
| | (0,066 kg/h) | 89,4 TBA |

Der erzielte Gesamt-Umsatz betrug: 87,0 %

### 2. Beispiel (Vergleich)

Die Stromnummern in der folgenden Tabelle 2 entsprechen denen in Fig. 1. Es sind Massenteile angegeben. Der Zulauf an Raffinatstrom (Raffinat I) betrug 0,3 kg/h. Es wurde ohne zusätzliche Verdampfung gearbeitet, um den Effekt auf den Umsatz zu untersuchen.

**Tabelle 2**

| Stromnummer | Strombezeichnung (Massenstrom) | Massen-Teile |
|---|---|---|
| 1 | Prozesswasser | 91,3 Wasser |
| | (0,052 kg/h) | 8,7 TBA |
| 9 | Bypass | |
| | (0 kg/h) | |
| 10 | Vor-Flash | 6, 8 Isobuten |
| | (0,415 kg/h) | 3,9 Wasser |
| | | 49,7 TBA |
| | | 39,5 Rest-C₄ |
| 12 | Flash-Raffinat | Siehe Strom Nr. 10, |
| | (0,415 kg/h) | kein Flash |
| 13 | Flash-TBA (0 kg/h) | Kein Flash |
| 20 | Rück-TBA | 10,4 Wasser |
| | (0,066 kg/h) | 89,4 TBA |
| 15 | Schlussreaktor- | 5,7 Isobuten |
| | Ausgang | 4,4 Wasser |
| | (0,419 kg/h) | 50,7 TBA |
| | | 39,1 Rest-C₄ |

Der erzielte Gesamt-Umsatz betrug: 82,4 %

### 3. Beispiel (gemäß der Erfindung)

Die Stromnummern in der folgenden Tabelle 3 entsprechen denen in Fig. 2. Es sind jeweils Massenteile angegeben. Der Zulauf an Raffinatstrom (Raffinat I) betrug 0,3 kg/h.

**Tabelle 3**

| Stromnummer | Strombezeichnung (Massenstrom) | Massen-Teile |
|---|---|---|
| 1 | Prozesswasser | 91,3 Wasser |
| | (0,051 kg/h) | 8,7 TBA |
| 9 | Bypass | |
| | (0 kg/h) | |
| 10 | Vorletzter Reaktor | 6,8 Isobuten |
| | (0,415 kg/h) | 3,9 Wasser |
| | | 49,7 TBA |
| | | 39,5 Rest-C₄ |
| 27 | Rückgeführtes | 10,7 Isobuten |
| | Destillat-Raffinat | 0,9 Wasser |
| | (0,280 kg/h) | 0,8 TBA |
| | | 87,7 Rest-C₄ |
| 29 | Recycle-TBA | 6,2 Wasser |
| | (0,164 kg/h) | 93,8 TBA |
| 20 | Rück-TBA | 10,4 Wasser |
| | (0,066 kg/h) | 89,4 TBA |
| 22 | Schlussreaktor- | 4,8Isobuten |
| | Ausgang | 2,7 Wasser |
| | (0,447 kg/h) | 37,5 TBA |
| | | 55,0 Rest-C₄ |

### Der erzielte Gesamt-Umsatz betrug: 85,4 %

### Vergleich der Beispiele:

Sowohl durch die einfache Verdampfung in Beispiel 1 (gemäß der Erfindung) als auch durch die neue Verschaltung gemäß Beispiel 3 (gemäß der Erfindung) ließ sich der Umsatz deutlich steigern im Vergleich zur herkömmlichen Nacheinanderschaltung der Reaktoren gemäß Beispiel 2 (Vergleichsbeispiel).

## Patentansprüche

1. Verfahren zur Herstellung von tert. Butanol (TBA) durch Umsetzung eines Gemisches, das Wasser, TBA und ein Isobuten-haltiges Kohlenwasserstoffgemisch aufweist, an einem sauren Katalysator in einer Reaktorkaskade, die zumindest zwei Reaktoren aufweist, bei 30 bis 120 °C, und anschließende destillative Abtrennung der nicht umgesetzten Kohlenwasserstoffe,
**dadurch gekennzeichnet,**
**dass** aus dem Reaktoraustrag zumindest eines Reaktors, der nicht der letzte der Reaktorkaskade ist, ein Teil des TBA abgetrennt wird, bevor der an TBA abgereicherte Reaktoraustrag in den nachfolgenden Reaktor geleitet wird.

2. Verfahren nach Anspruch 1,
**dadurch gekennzeichnet,**
**dass** die Abtrennung eines Teils des TBA aus dem Reaktionsaustrag des Reaktors, der nicht der letzte der Reaktorkaskade ist, durch Destillation bzw. Rektifikation erfolgt, wobei der an TBA abgereicherte Produktstrom in den nachfolgenden Reaktor geführt wird und der an TBA angereicherte Produktstrom zusammen mit dem Austrag des nachfolgenden Reaktors in eine Kolonne zur Aufarbeitung von Roh-TBA (Roh-TBA-Kolonne) eingespeist wird.

3. Verfahren nach Anspruch 1 und 2,
**dadurch gekennzeichnet,**
**dass** die Abtrennung eines Teils des TBA durch Elashung/einfache Verdampfung oder einfache Destillation erfolgt.

4. Verfahren nach Anspruch 1,
**dadurch gekennzeichnet,**
**dass** der Reaktoraustrag aus dem Reaktor, der nicht der letzte der Reaktorkaskade ist, ganz oder teilweise zusammen mit dem Austrag des letzten Reaktors in eine Kolonne zur Abtrennung von Roh-TBA (Roh-TBA-Kolonne) eingespeist wird und dass ein Teil des Kopfproduktes und ein Teil des Sumpfproduktes der Roh-TBA-Kolonne in den letzten Reaktor eingeleitet werden.

5. Verfahren nach einem der Ansprüche 1 bis 4,
**dadurch gekennzeichnet,**
**dass** die Abtrennung des TBA so erfolgt, dass das an TBA abgereicherte Produkt, welches in den nachfolgenden oder letzten Reaktor geführt wird, einen Gehalt an TBA von 10 bis 50 Massen-% aufweist.

6. Verfahren nach einem der Ansprüche 1 bis 4,
**dadurch gekennzeichnet,**
**dass** die Abtrennung des TBA aus einem Reaktorsaustrag zumindest eines Reaktors, der nicht der letzte der Reaktorkaskade ist, und der einen Gehalt an Isobuten kleiner 15 Massen-% aufweist, erfolgt.

## Claims

1. Process for preparing tert-butanol (TBA) by converting a mixture which comprises water, TBA and an isobutenic hydrocarbon mixture over an acidic catalyst in a reactor battery which has at least two reactors at from 30 to 120°C, and subsequently removing the unconverted hydrocarbons by distillation,
**characterized in that**
a portion of the TBA is removed from the reactor effluent of at least one reactor which is not the last reactor of the reactor battery before the TBA-depleted reactor effluent is passed into the subsequent reactor.

2. Process according to Claim 1,
**characterized in that**
the removal of a portion of the TBA from the reaction effluent of the reactor which is not the last of the reactor battery is effected by distillation or rectification, by conducting the TBA-depleted product stream into the subsequent reactor and feeding the TBA-enriched product stream together with the effluent of the subsequent reactor into a column for working up crude TBA (crude TBA column).

3. Process according to Claim 1 and 2,
**characterized in that**
the removal of a portion of the TBA is effected by flash evaporation/simple evaporation or simple distillation.

4. Process according to Claim 1,
**characterized in that**
the reactor effluent from the reactor which is not the last of the reactor battery is fed fully or partly, together with the effluent of the last reactor, into a column for removing crude TBA (crude TBA column) and a portion of the top product and a portion of the bottom product of the crude TBA column are introduced into the last reactor.

5. Process according to any of Claims 1 to 4,
**characterized in that**
the removal of TBA is effected in such a way that the TBA-depleted product which is conducted into the subsequent or last reactor has a content of TBA of from 10 to 50% by mass.

6. Process according to any of Claims 1 to 4,
**characterized in that**
the removal of TBA is effected from a reactor effluent of at least one reactor which is not the last of the reactor battery and has a content of isobutene of less than 15% by mass.

## Revendications

1. Procédé pour la production de tert-butanol (TBA) par mise en réaction d'un mélange qui comporte de l'eau, du TBA et un mélange d'hydrocarbures contenant de l'isobutène, sur un catalyseur acide dans une cascade de réacteurs qui comporte au moins deux réacteurs, à 30 à 120 °C, et séparation par distillation subséquente des hydrocarbures n'ayant pas réagi,
**caractérisé en ce qu'**on sépare une partie du TBA de la décharge de réacteur d'au moins un réacteur, qui n'est pas le dernier de la cascade de réacteurs, avant d'envoyer la décharge de réacteur appauvrie en TBA dans le réacteur suivant.

2. Procédé selon la revendication 1,
**caractérisé en ce que** la séparation d'une partie du TBA d'avec la décharge de réaction du réacteur, qui n'est pas le dernier de la cascade de réacteurs, s'effectue par distillation ou rectification, le courant de produit appauvri en TBA étant envoyé dans le réacteur suivant et le courant de produit enrichi en TBA étant injecté conjointement avec la décharge du réacteur suivant dans une colonne destinée au traitement final du TBA-brut (colonne pour TBA-brut).

3. Procédé selon la revendication 1 et la revendication 2,
**caractérisé en ce que** la séparation d'une partie du TBA s'effectue par vaporisation simple/par détente ou distillation simple.

4. Procédé selon la revendication 1,
**caractérisé en ce qu'**on injecte dans une colonne destinée à la séparation du TBA-brut (colonne pour TBA-brut) la décharge de réacteur provenant du réacteur, qui n'est pas le dernier de la cascade de réacteurs, en partie ou en totalité conjointement avec la décharge du dernier réacteur, et **en ce qu'**on envoie dans le dernier réacteur une partie du produit de tête et une partie du produit de bas de la colonne pour TBA-brut.

5. Procédé selon l'une quelconque des revendications 1 à 4,
**caractérisé en ce que** la séparation du TBA s'effectue par le fait que le produit appauvri en TBA, qui est envoyé dans le réacteur suivant ou dernier, présente une teneur en TBA de 10 à 50 % en masse.

6. Procédé selon l'une quelconque des revendications 1 à 4,
**caractérisé en ce que** la séparation du TBA s'effectue à partir d'une décharge de réacteur d'au moins un réacteur, qui n'est pas le dernier de la cascade de réacteurs, et qui présente une teneur en isobutène inférieure à 15 % en masse.
